# EUROPEAN PATENT APPLICATION

(11) **EP 4 830 923 A1**
(43) Date of publication of application: **28.10.2026**
(21) Application number: 25382415.5
(22) Date of filing: 24.04.2025
(51) Int. Cl.: B33Y 10/00, B29C 64/112, B29C 64/118, B33Y 80/00, C12M 1/12

(54) **A METHOD FOR 3-D PRINTING A SCAFFOLD FOR CELL CULTURE**

(71) Applicant: Fundacion Centro de Tecnologias de Interaccion Visual y comunicaciones Vicomtech, 20009 San Sebastian (ES); Asociación Centro de Investigación Cooperativa en Biomateriales - CIC biomaGUNE, 20014 San Sebastián (ES)
(72) Inventor: JIMENEZ DE ABERASTURI, Dorleta, 20014 San Sebastián (ES); AIZARNA, Uxue, 20014 San Sebastián (ES); HENRIKSEN, Malou, 20014 San Sebastián (ES); GARCÍA ASTRAIN, Clara, 20014 San Sebastián (ES); CORTÉS ACOSTA, Camilo Andrés, 20009 San Sebastián (ES); MEJÍA PARRA, Daniel, 20009 San Sebastián (ES); ZELAIA AMILIBIA, Maialen, 20009 San Sebastián (ES); BERASATEGI OÑATIBIA, Garat, 20009 San Sebastián (ES)
(74) Representative: Balder IP Law, S.L.

(57) **Abstract**

The application relates to a method of for 3-D printing a scaffold for cell culture comprising a first set of scaffold layers and additional sets of scaffold layers, each set comprising at least one layer, each layer comprising an infill portion defining a same pattern, and the infill portion of each layer overlapping the infill portions of the rest of the layers; the method comprising:
i) printing the first set;
ii) printing an additional set in contact with the most-recently printed set, wherein the infill portion of each layer of the additional set is printed forming the same angle of at least 10° and at most 80° with respect to the infill portion of the most-recently printed layer of the most-recently printed set; and
iii) repeating ii).

## Description

### TECHNICAL FIELD

The present invention is encompassed within the field of 3-D printing. More specifically, in the field of 3-D printing scaffolds for cell culture.

### BACKGROUND

A scaffold for cell culture is a structure to which cells attach. The cells grow and proliferate in vitro while being attached to the scaffold.

3-D printing (i.e., three-dimensional printing) refers to making a 3-D physical object by sequentially adding material to make the object. In some types of 3-D printing, the material may be added by deposition of material in determined locations, for example, by extrusion or by inkjet printing. After depositing the material, the material solidifies. In some types of 3-D printing (e.g., stereolithography or powder bed fusion) material to be subsequently solidified is readily deposited, so that the printing involves solidifying (e.g., by photo-polymerization or sintering) the material in determined locations. The deposition/solidification locations correspond to portions of the object to be printed and are usually determined based on a computer-readable model of the object. Subsequently added material binds to previously added material. The binding may be achieved by the solidification of subsequently added material when in contact with previously added material.

Usually, prior to performing the 3-D printing, a computer-readable model of the object to be printed is processed to generate instructions readable by a 3-D printer (more specifically, instructions to be executed by at least one computing device of a 3-D printer), the instructions being for 3-D printing the object. The model is, for example, a Computer-Aided-Design (i.e., CAD) file. The instructions are in a suitable format, for example, G-code.

To do so, a software commonly known as slicer is used to segment the model (i.e., to virtually slice the object) as a stack of the layers to be printed. The stack normally results from cutting the model by parallel virtual planes perpendicular to a given axis, normally the Z axis or height direction, at different heights or cutting points of the Z axis, the layers being, in general, parallel to each other. The layers are, in general, horizontal.

In types of 3-D printing that, like stereolithography or powder bed fusion, rely on solidifying determined portions of already deposited material, incidence location of a solidification agent (e.g., a light beam) is defined by the instructions. In types of 3-D printing that, like ink-jet printing and printing by extrusion, rely on controlling the location where material is deposited, a printing head deposits the material in locations defined by the instructions.

A material printed (e.g., deposited, for example, by extrusion) in 3-D printing is, for example, a hydrogel, for example, at least one of: agarose, alginate, chitosan, collagen, fibrin, gelatin, hyaluronic acid, polyethylene glycol and polyethylene glycol diacrylate.

The scaffold is printed layer by layer. It is known that an object to be printed (e.g., a scaffold) can be virtually sliced in horizontal layers, so that the object may be printed by consecutively printing the horizontal layers. Printing the layers horizontally and each layer on top of the previous one, provides mechanical support for subsequently printed layers. Each subsequent layer is printed in contact with a most-recently printed layer (e.g., on a most-recently printed layer), so that the most-recently printed layer supports the subsequent layer and, in most 3-D printing techniques, binds to the subsequent layer. In this way, the object may be 3-D printed by printing an initial layer (e.g., a lowermost layer, which can be considered to be at a height of zero, i.e., at a cutting point in the zero coordinate of the Z axis) supported by a horizontal structure and iteratively printing each layer in contact with the most-recently printed layer.

Each layer of the object may be completely printed prior to starting the printing of the subsequent layer. This allows minimizing the number of changes of height, i.e. positions at Z axis, of the printing head of the 3-D printer, and hence a minimization of printing time and displacements of printing head, resulting in optimized printing performance. This optimization results from all the portions of the object at a same height, i.e. in the same layer, being printed prior to moving the printing head, relatively to the printed layers, to another height, i.e. to another position in the Z axis, where no layer has been printed yet. In addition, completely printing each layer prior to starting to print the subsequent layer allows maximizing time available for layer solidification prior to printing the subsequent layer.

Each layer of a 3-D printed object may comprise one or more infill areas delimited by boundary areas.

The infill areas are areas in which an infill is 3-D printed. The infill may have different grades of porosity or void fraction, of more than zero and less than one hundred, have different designs -such as regular or irregular grid with different shapes- and may be made of the same or different material as the other areas of the 3-D printed object. In general the infill has the purpose of providing structural stability to the 3-D printed object and an appropriate support for each subsequent layer to be printed. Therefore, the infill, in general, is designed to minimize the use of material and printing time while ensuring sufficient mechanical strength of the 3-D printed object. The infill is configured to obtain a 3-D printed object with determined mechanical and functional properties. For example, the infill pattern and the orientations of the infill pattern may be configured to obtain a determined porosity. In the context of the present disclosure, the expression "porosity" when referring to an infill can be calculated with the following equation: $Porosity of the infill = \frac{Total volume of the infill - Filled volume of the infill}{Total volume of the infill} \times 100$

The boundary areas of a layer are optionally printed 3-D printed areas of a layer that surround the infill areas of a layer. Depending on desired functional and structural properties of the 3-D printed object, boundary areas may have different designs.

The purpose of some 3-D printed objects is to promote the adhesion and growth of cells. These 3-D objects are often called cell culture scaffolds. In cell culture scaffolds, boundary areas are optionally printed depending on the scaffold design and the infill has additional functions such as defining the porosity of the 3-D printed object to allow the penetration of some fluids, and providing a sieving effect to retain cultured cells which are seeded after printing the scaffold and prevent their falling to the bottom of the scaffold. However, the infill of cell culture scaffolds known in the state-of-the-art provide very small uniformity of distribution of cells throughout the entire height of the 3D-printed scaffolds. More specifically, pores of the infill are too big to hold the cells, so that most of the cells settle at the bottom of the scaffold. Thus, cell culture scaffolds which allow a more uniform distribution of cells are desirable. Therefore, there is a pressing need to develop cell culture scaffolds that provide an optimum design to retain cultured cells which can be present over all the different layers while providing access to culture medium and other fluids required during the processing of the cultured cells.

### SUMMARY

A first aspect of the disclosure relates to a method for 3-D printing a scaffold for cell culture comprising a first set of scaffold layers and additional sets of scaffold layers, each set comprising at least one layer, each layer comprising an infill portion defining a same pattern, and the infill portion of each layer overlapping the infill portions of the rest of the layers; the method comprising:
i) printing the first set;
ii) printing an additional set in contact with the most-recently printed set, wherein the infill portion of each layer of the additional set is printed forming the same angle of at least 10° and at most 80° with respect to the infill portion of the most-recently printed layer of the most-recently printed set; and
iii) repeating ii).

The pattern defined by the infill comprises void spaces. A problem of said void spaces is that when cells are deposited on said void spaces, the cells may not find a support required to proliferate or may find such support at a lowermost portion of the scaffold, so that a relatively large volume of the scaffold is not effectively used for cell culture. The present disclosure allows maximizing the proportion of scaffold volume used for cell culture by causing that the infill portion of each layer of the first set and of the additional sets overlaps the infill portion of the rest of the layers of the first set and the additional sets and by rotating the infill portions of the additional sets as indicated in the present disclosure. By printing the infill portions rotated at an angle of at least 10° and at most 80°, for example, of at least 15° and at most 75° or, for example, at least 20° and at most 70°, a pore shape of the scaffold is more staggered compared to rotating the infill portions by an angle of 90°. This happens because the rotation in each execution of ii) causes that there is scaffold material below void spaces of a scaffold layer, so that if cells go through the void space, chances that they are supported by the scaffold material below the void space are increased compared to not having scaffold material below the void space (e.g., due to a rotation of 90°). Therefore, uniformity of distribution of cells throughout the entire 3-D printed scaffold is enhanced compared to scaffolds not having scaffold material below the void spaces. This allows starting the cell culture with more cells than if the infill portions were not rotated or were rotated an angle of 90°.

In the present disclosure, it is considered that an infill portion of a layer overlaps an infill portion of another layer if both infill portions are, at least in part, in a same vertical. In other words, they overlap when the projection of the infill portion of a layer intersects the projection of the infill portion of another layer, the projections being generated by projection rays perpendicular to the layers (i.e., perpendicular to the planes virtually slicing the model) in a projection plane; the projection plane being perpendicular to the projection rays.

If the same additional set comprises more than one layer, the infill portion of each layer of the additional set forms a same angle with respect to the infill portion of the most-recently printed layer of the most-recently printed set. In other words, the infill portion of each layer of the same set has the same orientation.

Compared to angles of 90°, angles of rotation of infill portions of at most 80°, for example, of at most 75° or 70° provide a smaller void space between the rotated infill portions, which allows enhancing cell support.

Compared to angles of 0°, angles of rotation of infill portions of at least 10°, for example, of at least 15° or 20°, reduce space between the rotated infill portions, which allows enhancing cell support.

Compared to smaller angles, an angle of at least 10°, for example, of at least 15° or 20°, allows minimizing the number of repetitions of ii) required for the infill portions to achieve a complete rotation. The decreased number of repetitions of ii) allows decreasing height of the scaffold required for achieving a determined number of rotations of the infill portion.

In some embodiments, at least one of the additional sets is a stack of layers. Thereby, at least one set of layers comprises a plurality of layers wherein the infill portion of each layer has a same orientation. Providing a set comprising more than one layer allows increasing the height/thickness of the set.

In some embodiments, at least one of the additional sets is a set of a single layer. Providing at least one set comprising just one layer allows rotating the infill portion in the printing of each layer of each set comprising just one layer, so that more infill portion rotations are formed per volume unit compared to printing sets comprising more than one layer. In this way, fewer layers are required to achieve a particular number of rotations of infill portions, thus allowing to save scaffold material.

In some embodiments, wherein the angles formed in the executions of ii) have a same direction. In other words, the direction of the angles is one of clockwise and counterclockwise. The direction of the angles of different layers is determined from a same reference. Angles having a same direction allow simplifying the printing logic.

In some embodiments, the angles formed in the executions of ii) are of at most 40°. Angles of a maximum of 40° allow further limiting space between two consecutive rotated infill portions compared to greater angles hence allowing providing better cell support.

In some embodiments, the angle of each execution of ii) is of 30°. It has been observed that the angle of 30° provides better support than higher angles, e.g., than angles of 60°.

In some embodiments, ii) is repeated a number of times such that a result of summing the angle of each repetition of ii) is of at least 360°. This allows ensuring that the pattern has been rotated in total at least 360° so that at least one complete turn of the pattern is achieved.

To maximize the scaffold volume used effectively for cell culture by increasing height of the scaffold, ii) may be repeated further times so that, for example, the pattern is rotated in total an angle of at least Nx360° wherein N is of at least two, for example, at least eight or fifteen and of at most fifty, for example, at most forty or thirty.

In some embodiments, N is of at least between one and seven. It has been observed that between one and seven complete turns is in general the minimum number of turns, for the rotation angles of at least 10° and at most 80°, required to reach minimum potential sizes of the void spaces..

In some embodiments, adjacent parallel portions of filaments of the infill portion are at a distance of at least 0.2 mm and at most 1.5 mm from each other. The distance refers to the dimension of the void space between the adjacent parallel portions of filaments. This is a relatively small distance that can be achieved with 3-D printing and which, in general, is suitable for scaffolds for cell culture.

In some embodiments, the pattern comprises parallel straight lines. It has been observed that patterns comprising parallel straight lines (e.g., patterns consisting of parallel straight lines or patterns consisting of parallel and perpendicular straight lines) provide an appropriate support for cell culture. Each line of the pattern is, for example, a filament or a filament portion of the infill portion.

In some embodiments, parallel lines of the infill portion are made of a continuous filament.

In the present disclosure, the term "continuous" when referring to a filament refers to that the filament is continuous in space (i.e., the filament forms an unbroken whole). The fact that the filament is continuous is advantageous for enhancing cell support, and hence uniformity of distribution of cells throughout the scaffold, compared to scaffolds having relatively long discontinuities in the infill portions (i.e., relatively long interruptions of filament) because, compared to a filament interruption, the continuity of the filament provides an additional cell support, thus minimizing a number of cells that go through void spaces of the infill areas.

In some embodiments, the 3D-printing of the infills comprises depositing (e.g., by extrusion or inkjet printing) continuous filaments, and the deposition of the continuous filament may be performed by depositing the filament continuously in time (i.e., without stopping the printing of the filament of the infill portion from beginning to end of the printing of the filament). Accuracy of location of a printing end and of a printing start is lower than the accuracy of location of material printed continuously in time. Thereby, printing the filament continuously in time allows enhancing accuracy of the filament and hence of the cell support.

In some embodiments, the scaffold comprises a shared body and divergent bodies, the divergent bodies diverging from the shared body; wherein each layer comprises an additional infill portion defining a same pattern, the additional infill portion of each layer overlapping the additional infill portions of the rest of the layers; wherein a divergent body is made of the infill portions, and another divergent body is made of the additional infill portions; each divergent body being distant from the rest of divergent bodies; and wherein in ii) the additional infill portion of each layer of the additional set is printed forming an angle of at least 10° and at most 80° with respect to the additional infill portion of the most-recently printed layer of the most-recently printed set.

Scaffolds comprising divergent bodies at a distance from each other allow printing scaffolds having more complex shapes. For example, it allows printing scaffolds comprising irregular geometries (e.g., biomimetic shapes).

In some embodiments, the pattern of the infill portions is the same as the pattern of the additional infill portions.

In some embodiments, the pattern of the infill portions is different than the pattern of the additional infill portions. Different patterns allow more flexibility of the geometry of the 3-D printed scaffold.

In some embodiments, the 3-D printing is at least one of extrusion and ink-jet printing. For example, the 3-D printing is one of extrusion and ink-jet printing.

In some embodiments, the method further comprises preparing a cell culture by seeding cells in the scaffold.

When seeding the cells, the scaffold must be at an appropriate temperature for the seeded cells. For example, if the material of the scaffold is printed at a temperature that may damage the cells or disturb cell proliferation or growth, it may be advantageous that the scaffold cools before seeding the cells in the scaffold.

In some embodiments, the method comprises seeding the cells after the scaffold has been completely 3-D printed. These embodiments may simplify the 3-D printing of the scaffold compared to embodiments in which the cells are placed prior to the 3-D printing of the scaffold (e.g., the cells are within the scaffold material to be printed) because the placement of the cells does not need to be considered during the 3-D printing of the scaffold. For example, it does not need to be considered the temperature of the scaffold when placing the cells nor material properties for printing a material together with the cells. Since the properties of the scaffold material during the printing do not need to be appropriate for printing the cells together with the scaffold material, the scaffold material may be chosen to further decrease a size of void spaces formed in the infill portions of a layer of the scaffold.

In some embodiments, the material printed in ii) comprises cells for cell culture. Thereby, the 3-D printer prints the scaffold material together with cells. In this way, the cells are deposited at the same time as the scaffold is deposited by the 3-D printer. These embodiments may have certain limitations of structural properties of the scaffold because the printed material and the printing conditions must be suitable for not unduly damaging the cells (e.g., sterile and sufficiently mild printing conditions, and material properties appropriate for being printed together with the cells).

In some embodiments, the cells are placed by placing a material in which the cells are embedded. For example, by 3-D printing (e.g., by inkjet printing or by extrusion) the material in which the cells are embedded. Placing a material in which the cells are embedded allows adjusting properties of the material to the placement method compared to placing cells not embedded in any material.

In some embodiments, the method further comprises seeding cells for cell culture on the additional set printed in ii), the seeding of the cells being performed before a subsequent repetition of ii).

Seeding cells in such manner results in seeding cells after just some layers of the scaffold have been printed. Seeding cells in such manner promotes cell support in intermediate layers of the scaffold compared to if cells are added after finishing the scaffold because, after finishing the scaffold, cell access to intermediate layers of the scaffold may be hindered by layers which have been subsequently printed. Thereby, a more effective use of the volume of the scaffold may be achieved by seeding cells after printing just some layers.

In some embodiments, the seeding of cells for cell culture in the scaffold is repeated after printing additional sets in repetitions of ii) and before subsequently repeating ii). Thereby, for similar reasons, cell support in the scaffold portion which has already been printed is promoted.

In some embodiments, the scaffold is 3-D printed by extrusion. In general, extruding the scaffold material provides a scaffold having better structural properties than if the scaffold material is deposited by ink-jet printing.

In some embodiments, the cells are deposited by ink-jet printing.

In some embodiments, the method comprises curing scaffold layers. Some materials require being cured prior to being suitable for use as scaffold layers.

Crosslinking allows using printing materials having low viscosity while allowing enough printing accuracy and material strength. Materials having low viscosity are usually required to be able to print the cells together with the structural material of the scaffold.

In some embodiments, the method comprises curing by moving a curing device (e.g., a device emitting curing light) with respect to the curable scaffold layers. Moving the curing device allows using a curing device having a relatively small spotlight size because the scaffold portions to be cured can be reached by moving the curing device.

In some embodiments, the curing device follows trajectories that are the same as or parallel to the trajectories of the printing head of the 3-D printer. For example, the curing device is attached to the printing head. For example, the trajectories of the curing device may be the same as printing trajectories of the scaffold but at a different height. A curing device that follows the same trajectories as or parallel trajectories to the printing trajectories of the layers allows ensuring that the whole layer is cured without having to perform several passes of the curing device, since it allows that the light of the curing device reaches every position in which the printing head has printed material by performing a trajectory similar to the one of the printing head.

In some embodiments, the method comprises repeating ii) one or more times without curing, with the curing device, the additional sets printed in said repetitions of ii), the method comprising curing, with the curing device, the additional sets printed in said repetitions of ii) after completing said repetitions of ii). By simultaneously curing more than one layer after having printed more than one layer, a number of curing steps required for curing the scaffold may be minimized because several layers are simultaneously cured with the curing device.

In some embodiments, the 3-D printer is configured to adjust irradiation time of curing light based on whether cells have been previously placed in the scaffold, e.g., based on whether cells have been placed in the bottom of the scaffold. Irradiation of curing light applied to the scaffold may also reach cells which have been placed in layers below the layers to be cured with the light. Basing the irradiation time in said placement of cells allows decreasing negative effects on the cells that may be caused by the light reaching the cells. For example, the 3-D printer may be configured to decrease irradiation time of a layer if cells have been previously placed in the scaffold compared to if cells have not been previously placed in the scaffold.

A second aspect of the disclosure relates to a scaffold for cell culture printed using the method of the first aspect of the disclosure.

A third aspect of the disclosure relates to a computer program product configured to generate instructions that, when executed by a 3-D printer, cause the 3-D printer to perform the method of the first aspect of the disclosure.

In some embodiments, the printing trajectories defined by the computer program are not rotated by the 3-D printer to provide the angle of ii). In other words, the printing trajectories defined by the computer program product already include the angle of step ii).

Similar advantages as those described for the first aspect of the disclosure may also be applicable to the second and third aspects of the disclosure.

### BRIEF DESCRIPTION OF THE DRAWINGS

To complete the description and to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 shows a diagram of a method according to some embodiments of the disclosure.
Figure 2 shows an example of virtual slicing of a generic object to be printed.
Figure 3 diagrammatically shows a system in accordance with embodiments.
Figure 4 shows top views of rotated infill portions of a scaffold for cell culture in accordance with embodiments.
Figure 5 shows perspective views of rotated infill portions of a scaffold for cell culture in accordance with embodiments.
Figure 6A shows an enlarged view of a projection of rotated infill portions in accordance with embodiments.
Figure 6B shows an enlarged view of a void space between adjacent filament portions of an infill in accordance with embodiments.
Figure 7A is a photograph of a scaffold for cell culture in accordance with embodiments, in which the infill portions have been rotated angles of 30°.
Figures 8A and 9A are images, obtained by a confocal fluorescence microscope, of a scaffold for cell culture in accordance with embodiments, in which the infill portions have been rotated angles of 30°; wherein the cells have been seeded in the cell culture after printing the scaffold.

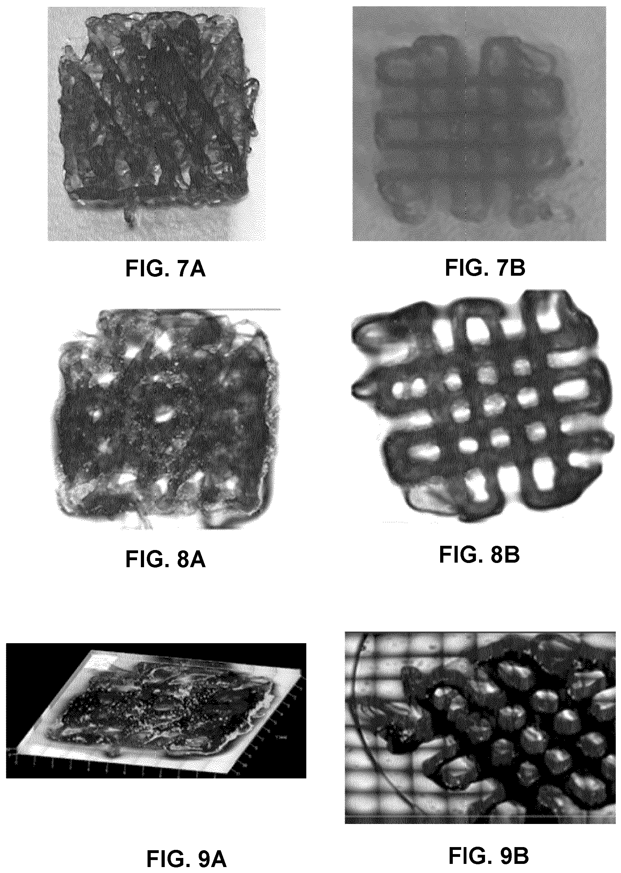

Figure 7B is a photograph of a scaffold for cell culture in which the infill portions have been rotated angles of 90° for performance comparison with the scaffold shown in figures 7A, 8A and 9A
Figures 8B and 9B show a scaffold for cell culture in which the infill portions have been rotated angles of 90° for performance comparison with the scaffold shown in figures 7A, 8A and 9A; wherein the cells have been seeded in the cell culture after printing the scaffold.

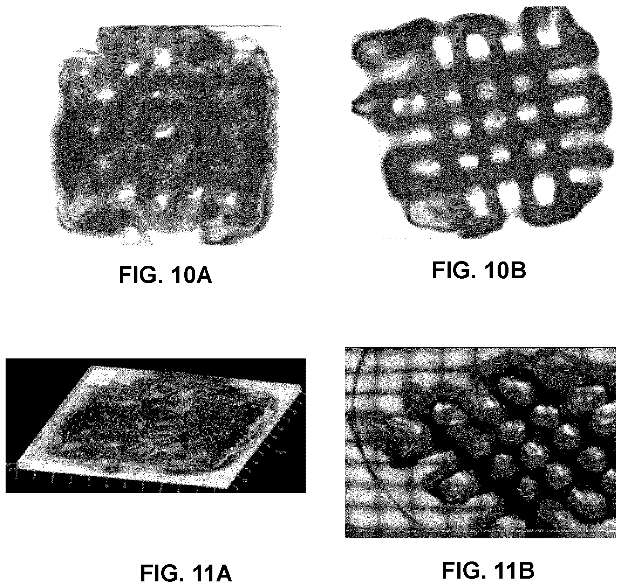

Figure 10A is the image of figure 8A in grayscale.
Figure 11A is the image of figure 9A in grayscale.
Figure 10B is the image of figure 8B in grayscale.
Figure 11B is the image of figure 9B in grayscale.

### DETAILED DESCRIPTION

The following description is not to be taken in a limiting sense but is given solely for the purpose of describing the broad principles of the invention. Embodiments of the invention will be described by way of example, with reference to the above-mentioned drawings.

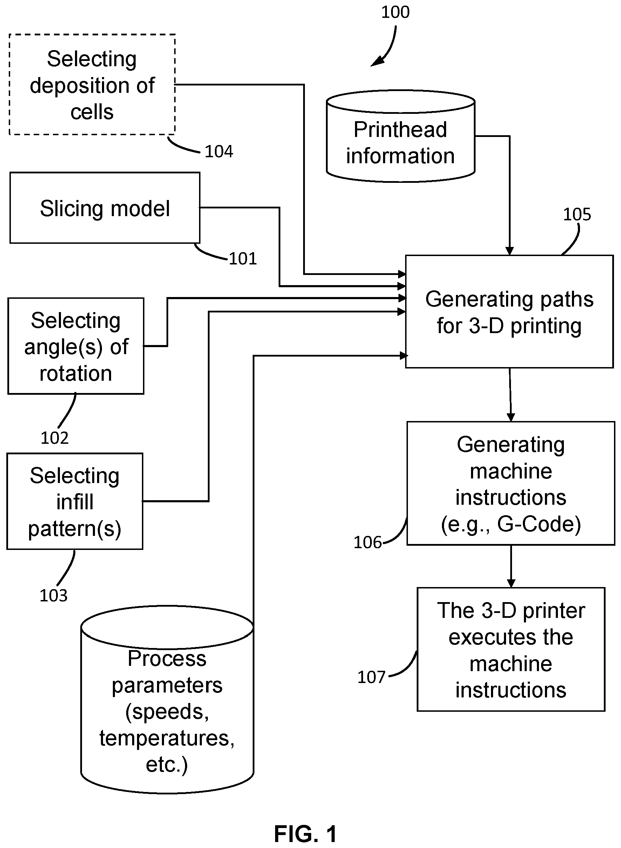

Figure 1 shows a diagram of an example of a method 100 in which instructions for operating a 3-D printer to print a scaffold for cell culture according to the present disclosure are generated and executed.

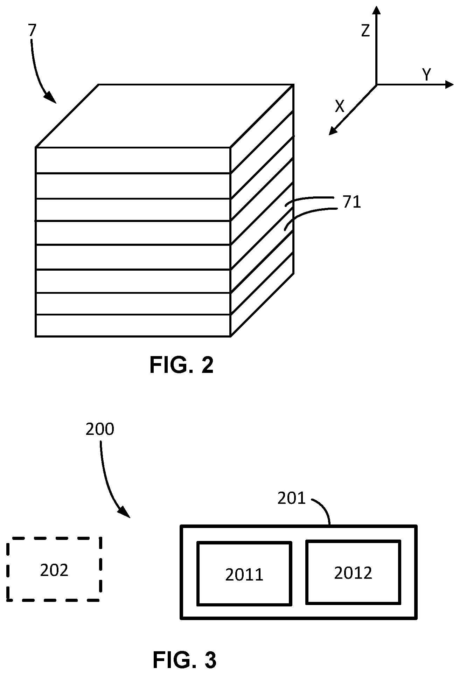

Methods according to the present disclosure can be performed with the system 200 shown in figure 3. The system 200 comprises a 3-D printer 201. The system 200 optionally comprises at least one computing device 202 external to the 3-D printer 201. The 3-D printer 201 comprises at least one computing device 2011 and 3-D printing hardware 2012. The 3-D printing hardware 2012 is communicatively connected with the at least one computing device 2011. The at least one computing device 2011 is configured to control de 3-D printing hardware 2012.

Steps of the method performed prior to the 3-D printing (i.e., prior to the execution of the machine instructions to print the scaffold) may be performed with the at least one computing device 2011 or with the at least one computing device 202.

The at least one computing device 2011 may comprise at least one of, for example, a CPU, a GPU, an FPGA, an ASIC, a personal computer and a laptop. The at least one computing device 202 may comprise at least one of, for example, a CPU, a GPU, an FPGA, an ASIC, a personal computer and a laptop.

The at least one computing device 2011 may be communicatively coupled with a data storage device, e.g., a data storage device of the 3-D printer 201.

The at least one computing device 202 may be communicatively coupled with at least one data storage device, e.g., a storage device external to the 3-D printer 201.

Although in figure 1, steps 101, 102, 103 and 104 have been illustrated as individual and separate steps, in some embodiments, said steps can be combined or further divided in substeps. In addition, in some embodiments, at least one of said steps is based on the output of another step (e.g., the selection of angles of rotation may be based on the selected infill pattern). More specifically, the at least one computing device 202, 2011 of the 3-D printer and/or external to the 3-D printer may be configured to perform at least one of steps 101, 102, 103 and 104 based on the output of another one of steps 101, 102, 103 and 104. For example, the at least one computing device 202, 2011 of the 3-D printer and/or external to the 3-D printer may be configured to select to an angle of rotation which is different than an angle of symmetry of the selected infill pattern.

The method 100 comprises slicing 101 a computer-readable model of the scaffold to be printed. An example of slicing of a computer-readable model of a generic object is shown in figure 2. The generic object is a rectangular cuboid 7. The rectangular cuboid has a height in the illustrated Z direction. The slicing involves virtually cutting the model at different heights of the model with planes, wherein the planes are perpendicular to the Z direction. Each plane separates one layer 71 of the model from an adjacent layer 71 of the model. In general, and as illustrated in figure 2, the Z direction is a vertical direction and the planes are horizontal planes (i..e, planes which are parallel to the X-Y plane).

The slicing 101 may be performed so that each one of the layers has the same height/thickness (i.e., the same dimension in the Z direction) or so that the layers have different heights.

The slicing 101 may provide data indicative of the external contours of each of the layers 71 of the scaffold, data indicative of the dimension of each layer in the Z direction 71, and data indicative of the location of each layer in space (e.g., of the printing order of the layers).

The method 100 comprises selecting 102 one or more angles of rotation of infill portions of sets of layers 71 of the scaffold. The selected angles are of at least 10° and at most 80°.

The method 100 comprises selecting 103 one or more infill patterns of the layers 71 of the scaffold.

The method 100 optionally comprises selecting 104 whether cells are placed in the scaffold after completing the 3-D printing of the scaffold or before completing the 3-D printing of the scaffold.

The selection may be performed by a user of the system 2 or automatically by at least one computing device 202, 2011. For example, the at least one computing device, 202, 2011 may be configured to select 102, 103, 104 a combination of angle(s) of rotation, infill pattern(s) and deposition of cells which are compatible with each other and with the model slicing.

The method 100 comprises generating paths for 3-D printing the scaffold based on the sliced model, on the selected angle(s) of rotation, on the selected infill pattern(s), on the selected deposition of cells and on the process parameters (e.g., temperature of printed material and/or printing speed). For example, if the 3-D printing is performed with a printhead (e.g., by extrusion or ink-jet printing) the process parameters may comprise data of the printhead, for example, size of the printhead and/or range of speed of deposition of material (e.g., range of extrusion speed). If the 3-D printing is performed by using a light source (e.g., stereolithography or laser sintering), the process parameters may comprise features of the light source (e.g., ranges of sizes of spots of light on the material to be 3-D printed and/or ranges of power of emitted light).

The method 100 comprises generating 106 machine instructions (e.g., G-code) based on the generated paths. The machine instructions are instructions which, upon execution by a 3-D printer, result in printing the scaffold by printing along paths generated in 105.

The method 100 comprises executing, by a 3-D printer, the machine instructions generated in 106.

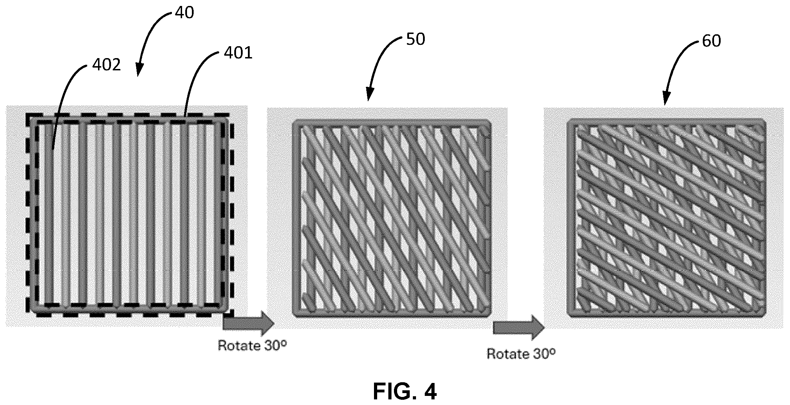

Figure 4 shows an example of 3-D printing three sets of layers of a scaffold, wherein each set of layers comprises just one layer. More specifically, figure 4 shows three layers: layer 40, layer 50 and layer 60, although the scaffold of figure 4 comprises more than three layers. Layer 40 can be considered the first set of layers, layer 50 can be considered an additional set of layers, and layer 60 can be considered an additional set of layers.

The infill of each portion has the same pattern, more specifically, a pattern of parallel straight lines.

Each layer comprises an infill area and a boundary area. The boundary area of each layer delimits the infill area of the same layer. Figure 4 shows the boundary area 401 and the infill area 402 of layer 40. Boundary area 401 is printed.

As shown in figure 4, layer 50, which is on top of layer 40 (i.e., the most-recently printed layer of the most-recently printed set when layer 50 begins to be printed), comprises an infill portion which forms an angle of 30° with respect to the infill portion of layer 40. The infill portion of layer 50 overlaps an infill portion of layer 40.

Figure 4 shows layer 60 on top of layer 50 (i.e., the most-recently printed layer of the most-recently printed set when layer 60 begins to be printed). Layer 60 comprises an infill portion which forms an angle of 30° with respect to the infill portion of layer 50. The infill portion of layer 60 overlaps an infill portion of layer 50 and the infill portion of layer 40.

Subsequent sets of layers of the scaffold can be deposited performing rotation of the infill portion of each additional set of layers by at least 10° and at most 80° (e.g., of 30°) with respect to the infill portion of the most-recently printed layer of the most-recently printed set.

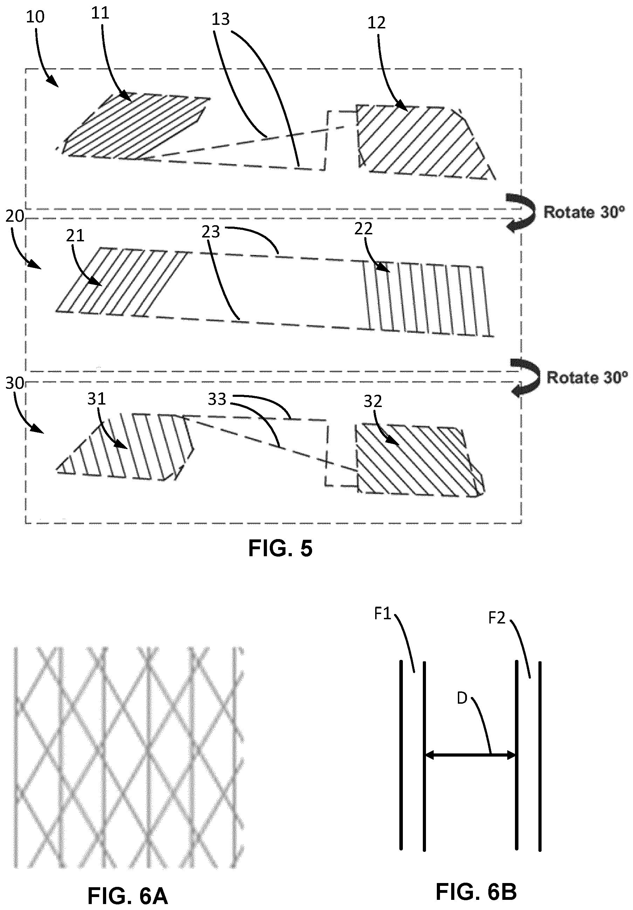

Figure 5 shows perspective views of paths of a printhead for 3-D printing three layers of a scaffold for cell culture.

Figure 5 shows in dashed lines 13, 23, 33 paths of the printhead along which no material is printed; and shows in continuous lines the paths of the printhead along which material is printed to generate infill portions.

Figure 5 shows that each layer comprises two infill areas. The infill areas shown in figure 5 have the same pattern. More specifically, a pattern of parallel straight lines. In each layer, the infill areas 11, 21, 31 are at a distance from the infill areas 12, 22, 32. Printing infill areas having distanced boundary areas in a same layer allows 3-D printing scaffolds having distanced divergent bodies, more specifically, scaffolds having shapes wherein several bodies diverge from a shared body, so that each one of the divergent bodies does not contact any other of the divergent bodies. The layers shown in figure 5 may correspond to two divergent bodies: one of the divergent bodies comprising infill areas 11, 21 and 31, and the other one of the divergent bodies comprising infill areas 12, 22 and 32.

An example of scaffold comprising bodies which diverge from a main body is a Y-shaped scaffold.

Figure 5 shows that layer 10 comprises infill area 11 and infill area 12, layer 20 comprises infill area 21 and infill area 22, and layer 30 comprises infill area 31 and infill area 32. Infill area 21 is deposited on top of infill area 11, and infill area 21 forms an angle of 30° with infill area 11. Infill area 31 is deposited on top of infill area 21, and infill area 31 forms an angle of 30° with infill area 21. Infill area 22 is deposited on top of infill area 12, and infill area 22 forms an angle of 30° with infill area 12. Infill area 32 is deposited on top of infill area 22, and infill area 32 forms an angle of 30° with infill area 22.

The rotations of 30° of the infill areas 21, 22 with respect to the infill areas 11, 12 are in the same direction as the rotations of 30° of the infill areas 31, 32 with respect to the infill areas 21, 22.

Figure 6A shows vertical projections (i.e., projections in the Z direction and hence perpendicular to the layers) in horizontal planes of portions of the infill areas 12, 22, 32. Figure 6A shows that the infill portions overlap because the infill portions are in a same vertical. In other words, the vertical projections of the infill portions in horizontal projection planes (i.e., projection planes perpendicular to the Z direction) intersect each other.

Figure 6B shows two contiguous filaments F1, F2 of an infill portion and a void space between the filaments F1, F2. The distance D between the filaments F1, F2 is of 0.5 mm.

Although in the embodiments shown in the figures each set of layers comprises just one layer, in other embodiments each set of layers comprises more than one layer. For example, in figure 4 instead of printing just one layer 40, more layers 40 (i.e., layers which have the same shape as layer 40 and the same X and Y coordinates as layer 40) may be printed to increase dimension in the Z direction of the shape of layer 40. Then several layers 50 (i.e., layers which have the same shape as layer 50 and the same X and Y coordinates as layer 40) may be printed on top of the layers 40. Then several layers 60 may be printed on top of the layers 50. Cells may be placed in the scaffold using cell placement methods known in the art.

Figures 7A to 9B show a comparison of experimental results between:
- a cell culture in a scaffold, shown in figures 7A, 8A and 9A, comprising infill portions which form angles of 30° (i.e., the angle of at least 10° and at most 80°); and
- a cell culture in a scaffold, shown in figures 7B, 8B and 9B, comprising infill portions which, instead of forming the angles of at least 10° and at most 80°, form angles of 90°.

Figures 8A and 8B show top views of the respective scaffolds. Figures 9A and 9B show lateral views of the respective scaffolds. Cells were placed in both scaffold using a same cell placement method. Cells are as shown in green dots.

As shown in figures 8A to 9B, there are more cells in the top and in the lateral side of the scaffold of figures 8A and 9A than in the top and in the lateral of the scaffold of figures 8B and 9B. Therefore, in the scaffold of figures 8A and 9A less cells fall to the bottom of the scaffold than in the scaffold of figures 8B and 9B.In the context of the present disclosure, and for the sake of conciseness, the feature "ii) printing an additional set in contact with the most-recently printed set, wherein the infill portion of each layer of the additional set is printed forming the same angle of at least 10° and at most 80° with respect to the infill portion of the most-recently printed layer of the most-recently printed set" is referred to as "ii)".

Grayscale figures 10A, 11A, 10B and 11B have been included to try to keep more details of the cell culture of figures 8A, 8B, 9A and 9B in case figures 8A, 8B, 9A and 9B are converted to black and white and more details are lost. In figures 10A, 10B and 11B, the green details of figures 8A, 8B and 9B are depicted as a dispersion of whitish points; and in figure 11A the green details of figure 9A are depicted as a dispersion of whitish points and as whitish lines (the biggest line being a whitish line in the right side of the scaffold).

In the context of the present disclosure, unless otherwise stated, the expression "infill portion" refers to a portion of an "infill area".

In the context of the present disclosure, unless otherwise stated, the expression "first set", when referring to the first set of scaffold layers, does not necessarily refer to the set of layers of the scaffold that is initially printed, but is merely used to distinguish the "first set" from the "additional sets". For example, the first set may be printed after printing some scaffold layers. However, in some embodiments, the "first set" refers to the set of scaffold layers which is initially printed.

In the context of the present disclosure, unless otherwise stated, the expression "most-recently printed set" refers to the additional set of layers or the first set of layers that has been completely printed the latest.

In the context of the present disclosure, unless otherwise stated, the expression "most-recently printed layer" refers to the layer, of any one of the additional sets or the first set, which has been completely printed the latest.

In the context of the present disclosure, unless otherwise stated, the expressions "porosity" and "void fraction" when referring to the porosity/void fraction of an infill refers to a proportion between void space of the infill and solid space of the infill.

In the context of the present disclosure, unless otherwise stated, the expression "void space" refers to a space which does not comprise solid material and hence does not comprise the printed material of the scaffold, so that liquid and/or gas can go through the void space.

In the context of the present disclosure, unless otherwise stated, the term "additional" when referring to an infill portion is used to distinguish the additional infill portions from the infill portions.

In the context of the present disclosure, unless otherwise stated, the term "additional" when referring to a boundary area is used to distinguish the additional boundary area from the boundary area.

In the context of the present disclosure, the term "comprises" and its derivations (such as "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A method for 3-D printing a scaffold for cell culture comprising a first set of scaffold layers and additional sets of scaffold layers, each set comprising at least one layer, each layer comprising an infill portion defining a same pattern, and the infill portion of each layer overlapping the infill portions of the rest of the layers; the method comprising:
i) printing the first set;
ii) printing an additional set in contact with the most-recently printed set, wherein the infill portion of each layer of the additional set is printed forming the same angle of at least 10° and at most 80° with respect to the infill portion of the most-recently printed layer of the most-recently printed set; and
iii) repeating ii).

2. The method of claim 1, wherein at least one of the additional sets is a stack of layers.

3. The method of any one of the previous claims, wherein at least one of the additional sets is a set of a single layer.

4. The method of any one of the previous claims, wherein the angles formed in the executions of ii) have a same direction.

5. The method of claim 4, wherein the angles formed in the executions of ii) are of at most 40°.

6. The method of any one of the previous claims, wherein ii) is repeated a number of times such that a result of summing the angle of each repetition of ii) is of at least 360°.

7. The method of any one of the previous claims, wherein the pattern comprises parallel straight lines.

8. The method of any one of the previous claims, wherein parallel lines of the infill portion are made of a continuous filament.

9. The method of any one of the previous claims, wherein the scaffold comprises a shared body and divergent bodies, the divergent bodies diverging from the shared body; wherein each layer comprises an additional infill portion defining a same pattern, the additional infill portion of each layer overlapping the additional infill portions of the rest of the layers; wherein a divergent body is made of the infill portions, and another divergent body is made of the additional infill portions; wherein each divergent body is distant from the rest of divergent bodies; and
wherein in ii) the additional infill portion of each layer of the additional set is printed forming an angle of at least 10° and at most 80° with respect to the additional infill portion of the most-recently printed layer of the most-recently printed set.

10. The method of any one of the previous claims, wherein the 3-D printing is at least one of extrusion and ink-jet printing.

11. The method of any one of the previous claims, further comprising preparing a cell culture by seeding cells in the scaffold.

12. The method of any one of the previous claims, wherein the material printed in ii) comprises cells for cell culture.

13. The method of any one of the previous claims, further comprising seeding cells for cell culture on the additional set printed in ii), the seeding of the cells being performed before a subsequent repetition of ii).

14. A scaffold for cell culture printed using the method of any one of claims 1 to 10.

15. A computer program product configured to generate instructions that, when executed by a 3-D printer, cause the 3-D printer to perform the method of any one of claims 1 to 10.
